# EUROPÄISCHE PATENTANMELDUNG

(11) **EP 2 248 544 A1**
(43) Veröffentlichungstag der Anmeldung: **10.11.2010**
(21) Anmeldenummer: 09075221.3
(22) Anmeldetag: 05.05.2009
(51) Int. Cl.: A61M 1/10

(54) **Im Durchmesser veränderbare Fluidpumpe, insbesondere für die medizinische Verwendung**

(71) Anmelder: ECP Entwicklungsgesellschaft mbH, 12247 Berlin (DE)
(72) Erfinder: Liebing, Reiner, 12587 Berlin (DE)
(74) Vertreter: Pfenning, Meinig & Partner GbR

(57) **Zusammenfassung**

Die Erfindung bezieht sich auf eine Fluidpumpeneinrichtung, insbesondere für die medizinische Verwendung, mit komprimierbarem Pumpengehäuse (6) und Rotor (7) sowie mit einem in einer Hülle (4) verlaufenden Betätigungsmittel (8), an dessen Ende die Fluidpumpe angeordnet ist. Um alle Möglichkeiten einer Platz sparenden Anordnung des jeweils für sich komprimierbaren Pumpengehäuses des Rotors und gegebenenfalls einer Lageranordnung (25) zu nutzen, sind die genannten Elemente in axialer Richtung gegenüber der Betriebsstellung gegeneinander verschiebbar. Insbesondere können diese Elemente nach der Montage durch eine Axialbewegung der Antriebswelle endkonfiguriert werden.

## Beschreibung

Die Erfindung liegt auf dem Gebiet der Mechanik, insbesondere der Feinwerktechnik, und ist mit besonderem Vorteil auf dem medizinischen Gebiet einsetzbar.

Unabhängig von dem Einsatz im medizinischen Sektor sind jedoch auch Anwendungen in anderen Gebieten dort vorstellbar, wo eine Fluidpumpe unter beengten Raumverhältnissen oder an schlecht zugänglichen Stellen betrieben werden soll.

Besonders trifft dies allerdings für die minimalinvasive Medizintechnik zu, wo häufig unter möglichst schonender Behandlung der Patienten medizinische Instrumente oder Geräte beispielsweise durch Blutgefäße an einen Einsatzort gebracht werden müssen. Insbesondere ist in diesem Zusammenhang der Einsatz von Blutpumpen in Zusammenhang mit Kathetern bekannt geworden, die beispielsweise in eine Herzkammer zur Unterstützung der Herzpumptätigkeit eingebracht werden können.

Da für eine optimierte Leistungsfähigkeit einer solchen Pumpe eine gewisse Größe notwendig ist, diese jedoch durch den Durchmesser der großen, im Herz endenden Blutgefäße des Körpers begrenzt ist, ist es bereits bekannt, im Radius veränderbare Fluidpumpen für diesen Zweck einzusetzen, die nach dem Verbringen in die Herzkammer expandiert werden können.

Ermöglicht wird dies entweder durch spezielle Mechanismen, die eine Betätigung eines Aufspannmechanismus der Pumpe durch einen Katheter ermöglichen oder durch die Verwendung von sogenannten Gedächtnismaterialien, die beim Ändern der Umgebungstemperatur verschiedene Formen annehmen können und durch eine Temperaturänderung in die gewünschte Endform gebracht werden.

Der Expandierbarkeit solcher Pumpen ist jedoch durch die Notwendigkeit, auf geringem Durchmesser sowohl eine Antriebswelle als auch einen Rotor und ein Pumpgehäuse unterzubringen, enge Grenzen gesetzt.

Der vorliegenden Erfindung liegt in diesem Zusammenhang die Aufgabe zugrunde, eine Fluidpumpe zu schaffen, die mit möglichst geringem konstruktivem Aufwand eine möglichst einfache und große Veränderbarkeit des Durchmessers erlaubt.

Die Aufgabe wird gemäß der Erfindung mit den Merkmalen des Patentanspruchs 1 gelöst.

Dabei ist ein im Durchmesser veränderbares Pumpengehäuse sowie gegebenenfalls auch ein im Durchmesser veränderbarer Rotor mit wenigstens einem Förderelement für das Fluid vorgesehen sowie ein in einer Hülle verlaufendes Betätigungsmittel,insbesondere ein Zugmittel, an dessen distalem Ende, von der Einbringungsstelle des Katheters aus gesehen, die Fluidpumpe angeordnet ist.

Das Betätigungsmittel ist in einer Längsrichtung verschiebbar. Um ein effizientes Komprimieren auf kleinen Durchmesser zu erlauben, sind das Pumpengehäuse und der Rotor durch das Betätigungsmittel in der Längsrichtung relativ zueinander verschiebbar. Dies kann dadurch realisiert sein, dass entweder das Pumpengehäuse oder der Rotor oder beide gegenüber dem Betätigungsmittel verschiebbar sind.

Beispielsweise kann für eine effiziente Komprimierung der Rotor wenigstens teilweise aus dem Pumpengehäuse herausbewegt werden. Die Kompressionsbewegung des Pumpengehäuses ist dann nicht durch den in ihm vollständig untergebrachten Rotor begrenzt.

Das Betätigungsmittel kann beispielsweise als Zugmittel genutzt werden und als Antriebswelle ausgebildet sein, an deren proximalem Ende ein Rotationsantrieb für die Pumpe vorgesehen ist. Die Antriebswelle ist in diesem Fall drehbar und zudem in Längsrichtung verschiebbar.

Neben dieser Ausführungsform besteht auch die Möglichkeit, die Pumpe mittels eines in den Körper implantierbaren am distalen Ende der Hülle angeordneten Miniaturmotors oder auch einer hydraulischen Mikroturbine anzutreiben. Anstelle der Antriebswelle verfügt die Fluidpumpe dann über ein geeignetes Betätigungsmittel wie beispielsweise einen Zug oder einen Draht oder ähnliches, welches den Rotor oder andere Teile der Pumpe verschiebt, insbesondere auch eine Lageranordnung in das Pumpengehäuse hineinzieht.

Es besteht zudem auch im erstgenannten Fall die Möglichkeit, zusätzlich zu einer Antriebswelle ein besser geeignetes Zugmittel zu verwenden. Dies erlaubt es, die Antriebswelle bezüglich der Anforderungen hinsichtlich Drehmomentübertragung und Laufzeit zu optimieren und das Zugmittel hinsichtlich der Zugfunktion unabhängig davon zu optimieren.

Ein solches Zugmittel kann beispielsweise ein Seil aus Kunststoff oder ein Drahtseil aber auch ein Draht oder jedes andere geeignete Zugmittel sein. Im Falle der Verwendung eines implantierbaren Miniaturelektromotors könnte man beispielsweise auch die zum Betrieb des Motors erforderlichen Kabel als Zugmittel verwenden, und im Falle der Verwendung einer hydraulischen Mikroturbine können die zum Betrieb dieser Turbine erforderlichen Hydraulikleitungen bzw. -schläuche als zugmittel verwendet werden.

Ist das Betätigungsmittel steif genug, beispielsweise als Draht ausgebildet, so kann auch eine Schubbewegung kontrolliert ausgeführt werden. Damit kann beispielsweise auch das Pumpengehäuse in Längsrichtung des Betätigungsmittels auf den Rotor zu geschoben werden.

Das Pumpengehäuse kann beispielsweise aus einem elastischen Gerüst, z. B. aus einer Gedächtnislegierung oder einem Kunststoff, bestehen, das mit einer Membran, beispielsweise aus Polyurethan beschichtet ist. Das Pumpengehäuse kann andererseits aber auch aus gegeneinander beweglichen Segmenten wie z. B. Schuppen oder Lamellen bestehen, die insgesamt beweglich und komprimierbar sind, wobei einzelne Schuppen/Segmente oder Lamellen in sich steif oder biegsam sein können. Der Rotor ist gegebenenfalls für sich komprimierbar dadurch, dass entweder einzelne Förderelemente des Rotors zur Verringerung des Durchmessers an die Welle anklappbar oder schwenkbar sind oder dass der Rotor aus einer durch ein oder mehrere Spannelemente aufspannbaren Membran besteht.

Zusätzlich zu den oben geschilderten Ausführungsformen des Pumpengehäuses und des Rotors sind auch andere komprimierbare und expandierbare Bauformen für den Einsatz der Erfindung denkbar.

Vorteilhaft ist für eine besonders gute Komprimierbarkeit der Anordnung, dass das Pumpengehäuse und der Rotor so weit in Längsrichtung des Betätigungsmittels gegeneinander verschiebbar sind, dass sie in der Längsrichtung hintereinander angeordnet werden können oder mit einer gegenseitigen Überlappung in Längsrichtung, die geringer ist als die Überlappung während des Betriebs der Fluidpumpe. Die verschiedenen Teile können dabei auf einer Antriebswelle verschiebbar sein, die entweder das Betätigungsmittel bildet oder dessen Verlängerung im Bereich der Pumpe ist, wenn am Ende der Hülle ein implantierbarer Miniaturmotor vorgesehen ist.

Eine besonders vorteilhafte Ausführungsform der Erfindung sieht zudem vor, dass am distalen Ende des Betätigungsmittels oder seiner Verlängerung, vom Rotationsantrieb aus gesehen hinter dem Rotor eine Lageranordnung auf dem Betätigungsmittel oder seiner Verlängerung angeordnet ist.

Ein besonders ruhiger und stabiler Lauf des Rotors wird dadurch erreicht, dass, insbesondere zusätzlich zu einer Lagerung am proximalen Ende des Pumpengehäuses, eine weitere Lagerung am distalen Ende des Pumpengehäuses vorgesehen ist. Die entsprechende Lageranordnung kann im Rahmen der Erfindung ebenfalls relativ zu dem Pumpengehäuse beweglich sein. Sie ist vorteilhaft auf dem Betätigungsmittel oder seiner Verlängerung in dessen/deren Längsrichtung verschiebbar oder zumindest mit dem Betätigungsmittel oder der Verlängerung gegenüber dem Rotor und dem Pumpengehäuse verschiebbar, sofern das Betätigungsmittel selbst in Längsrichtung verschiebbar ist.

Hierdurch kann nach dem Expandieren der Fluidpumpeneinrichtung die Lageranordnung durch eine weitere Verschiebungsbewegung an ihren Einsatzort am distalen Ende des Pumpengehäuses gebracht werden.

Eine weitere vorteilhafte Ausgestaltung der Erfindung sieht vor, dass die Lageranordnung in Längsrichtung des Betätigungsmittels relativ zum Rotor verschiebbar ist.

Damit können sowohl die Lageranordnung als auch der Rotor unabhängig voneinander komprimiert und expandiert und erst nach dem Anbringen der Fluidpumpeneinrichtung in Längsrichtung gegeneinander verschoben werden, um die Betriebsanordnung zu erreichen.

Wenn die Lageranordnung auf dem Betätigungsmittel oder der Verlängerung axial verschiebbar ist, dann sollte auf diesem, beispielsweise an dessen/deren Ende, ein Anschlagskörper vorgesehen sein, der beim Zurückziehen des Betätigungsmittels in Richtung der Betätigungsvorrichtung die Lageranordnung mitnimmt. Die Lageranordnung ihrerseits kann dann gegen den Rotor stoßen und auch diesen in Richtung des Pumpengehäuses bis zur Endposition bewegen.

Es kann dann zudem vorgesehen sein, dass die Lageranordnung mit dem Rotor in das Innere des Pumpengehäuses bewegbar ist und dass sternförmig angeordnete Streben vorgesehen sind, die sich zwischen einer Nabe der Lageranordnung und dem Pumpengehäuse elastisch verspannen.

Dies kann besonders vorteilhaft dadurch erreicht werden, dass die Streben elastisch schwenkbar an der Nabe der Lageranordnung befestigt sind.

Zu diesem Zweck können die Streben ebenso wie Lageranordnung beispielsweise aus einem elastischen Kunststoff oder Gummi bestehen oder aus einem federelastischen Metall.

Es kann auch vorteilhaft vorgesehen sein, dass die Streben durch axiale Kompression der Lageranordnung im Zuge der Bewegung der Antriebswelle durch Auffalten radial ausgefahren werden. Dies ist z. B. dann möglich, wenn die Lageranordnung zwei gegeneinander in Axialrichtung verschiebbare Ringe aufweist, zwischen denen die Streben befestigt sind, wobei die Streben bei einem größeren Abstand der Ringe flach anliegen und beim Zusammenschieben der Ringe wulstartig expandiert werden.

Die Streben können im verspannten Zustand vorteilhaft einen Einströmkäfig am distalen Ende der Fluidpumpe bilden, der einerseits verhindert, dass der Rotor mit Körpergewebe in Verbindung kommt, andererseits dafür sorgt, dass etwa in dem zu fördernden Fluid befindliche größere koagulierte Massen nicht in das Pumpengehäuse eindringen können.

Vorteilhaft ist außerdem, das Betätigungsmittel gegenüber der Hülle in Längsrichtung, insbesondere auf den Rotationsantrieb am proximalen Ende der Hülle zu, verschiebbar auszugestalten, um die verschiedenen axialen Verschiebungsbewegungen von Pumpengehäuse, Rotor und Lageranordnung gegeneinander zu bewirken.

Die Hülle ist dabei im Bereich der medizinischen Anwendung üblicherweise als Katheter ausgebildet. Ein solcher Katheter ist zwar biegsam, jedoch so steif, dass er durch ein Blutgefäß geschoben werden kann. Der Katheter ist üblicherweise mit dem Pumpengehäuse fluiddicht verbunden, wobei das Betätigungsmittel, beispielsweise eine Antriebswelle, möglichst dicht durch eine Drehdurchführung in das Pumpengehäuse eingeführt ist.
Am proximalen Ende, das üblicherweise außerhalb des Patientenkörpers liegt, ist der Katheter fluiddicht mit einer Betätigungsvorrichtung und beispielsweise auch mit einem elektromotorischen Antrieb verbunden, soweit nicht ein implantierter Mikromotor im Bereich der Fluidpumpe bevorzugt wird.
Vorteilhaft wird der Katheter mit einer körperverträglichen Flüssigkeit wie beispielsweise Kochsalzlösung gefüllt, um einerseits unbedingt das Eindringen von Gasblasen in den Körper zu verhindern und andererseits gegebenenfalls die Welle zu schmieren und zu kühlen, die sich üblicherweise mit 20.000-35.000 Umdrehungen pro Minute bewegt.

Die vorliegende Erfindung kann in dem Fall, dass innerhalb der Hülle eine Antriebswelle vorgesehen ist, weiter vorteilhaft dadurch ausgestaltet sein, dass das antriebsseitige Ende der Antriebswelle mit einem in einem dichten Gehäuse angeordneten, von außerhalb des Gehäuses magnetisch rotatorisch antreibbaren Antriebskörper verbunden ist.

Diese Ausführungsform erlaubt den Antrieb der Antriebswelle ohne eine abzudichtende Drehdurchführung, durch ein magnetisch inaktives Gehäuse hindurch, dadurch, dass ein Rotationsfeld angelegt wird, das den im Gehäuse befindlichen Antriebskörper und damit die Antriebswelle in Drehung versetzt.

Eine Verschiebbarkeit der Antriebswelle in Längsrichtung wird gemäß der Erfindung dadurch erreicht, dass der Antriebskörper in Längsrichtung der Antriebswelle verschiebbar ist und mantelseitig durch ein veränderliches magnetisches Feld angetrieben wird.

Durch die mantelseitige Übertragung der Antriebskräfte ist diese unabhängig von einer Axialverschiebung des Antriebskörpers beim Ziehen oder Schieben der Welle.

Es kann jedoch auch vorgesehen sein, dass die Antriebswelle fest mit einem Mitnahmekörper verbunden ist, der seinerseits in Längsrichtung der Antriebswelle verschiebbar direkt in dem Rotor geführt ist.

In diesem Fall ist der Antriebskörper in axialer Richtung der Antriebswelle ortsfest angeordnet, und lediglich ein Mitnahmekörper, der drehfest mit dem Rotor verbunden ist, ist seinerseits axial verschiebbar mit der Antriebswelle. Ein solcher Mitnahmekörper kann beispielsweise als im Querschnitt polygonförmiger, beispielsweise Achteckkörper ausgebildet sein. Die Erfindung bezieht sich außer auf eine Fluidpumpeneinrichtung der eingangs genannten Art auch auf ein Verfahren zum Betrieb einer derartigen Einrichtung, wobei vorgesehen ist, dass die Fluidpumpeneinrichtung in komprimiertem Zustand an einen Einsatzort gebracht wird, dass danach wenigstens das Pumpengehäuse wenigstens teilweise expandiert und dass danach das Pumpengehäuse und der Rotor derart in Längsrichtung eines Betätigungsmittels, insbesondere einer Antriebswelle des Rotors gegeneinander verschoben werden, dass der Rotor vollständig in dem Pumpengehäuse aufgenommen wird.

In den genannten Verfahrensschritten, nämlich dass zunächst die einzelnen Elemente Pumpengehäuse, Rotor und Lageranordnung an den Einsatzort gebracht, dort wenigstens teilweise expandiert und erst danach durch relative axiale Verschiebung in die zum Betrieb erforderliche Konstellation gebracht werden, ist eine stärkere Komprimierung der einzelnen Teile durch ihre Staffelung in Längsrichtung des Betätigungsmittels möglich.

Nach dem Einbringen der Anordnung beispielsweise in einen Patientenkörper kann mit den Mitteln der Erfindung beispielsweise die Antriebswelle zurückgezogen und hiermit sowohl die Lageranordnung als auch der Rotor in Richtung auf das Pumpengehäuse bewegt werden, bis dass der Rotor sich gänzlich im Pumpengehäuse befindet und durch die Lageranordnung gegebenenfalls abgestützt ist.

Ob der Rotor expandiert wird, bevor oder nachdem er in das Pumpengehäuse eingebracht ist, bleibt dabei offen, ebenso wie die Möglichkeit, das Pumpengehäuse zunächst vor dem Einbringen des Rotors nur teilweise zu expandieren, so weit, wie dies nötig ist, um den Rotor einzuführen, oder ob das Pumpengehäuse bereits vor dem Einführen des Rotors vollständig expandiert wird.

Im Folgenden wird die Erfindung anhand eines Ausführungsbeispiels in einer Zeichnung gezeigt und anschließend beschrieben. Dabei zeigt:
- Fig. 1: eine schematische Übersicht über eine Flu- idpumpeneinrichtung, wobei die Pumpe in ei- ne Herzkammer eingeschoben ist,
- Fig. 2: eine dreidimensionale Abbildung eines Pum- penrotors,
- Fig. 3: eine Seitenansicht des Pumpengehäuses, des Rotors und einer Lageranordnung,
- Fig. 4: eine Seitenansicht des Pumpengehäuses mit darin befindlichem Rotor,
- Fig. 5: ein Detail des Pumpengehäuses in dreidimen- sionaler Darstellung,
- Fig. 6: eine Lageranordnung in einer Seitenansicht,
- Fig. 7: die Lageranordnung aus Fig. 6 in einer Frontansicht,
- Fig. 8: eine weitere Lageranordnung,
- Fig. 9: eine Lageranordnung wie aus Fig. 8 in komp- rimiertem Zustand,
- Fig. 10: eine weitere Lageranordnung in einer Seitenansicht,
- Fig. 11: das proximale Ende einer Antriebswelle mit einer Ankopplung an einen Rotationsantrieb sowie
- Fig. 12: eine andere Ausgestaltung des proximalen Wellenendes mit einer anderen Ankopplung an einen Antrieb.

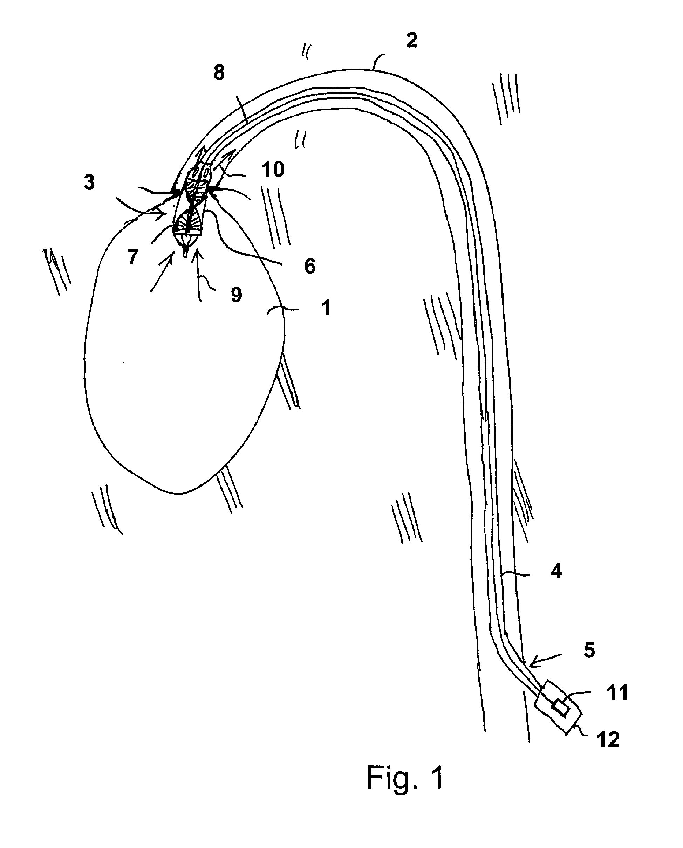

Fig. 1 zeigt eine Herzkammer, die mit einem Blutgefäß 2 verbunden ist, in das Blut gepumpt werden soll. Zur Unterstützung der Pumptätigkeit ist eine Fluidpumpe 3 in die Herzkammer 1 eingeführt, die dort Blut ansaugt und dies in das Blutgefäß 2 pumpt.

In das Blutgefäß 2 ist ein Katheter 4 durch eine Schleuse 5 eingeführt, durch die der Katheter auch wieder herausgezogen werden kann. Der Katheter 4 trägt an seinem distalen Ende die Pumpe 3 in Form eines mit dem Katheter verbundenen Pumpengehäuses 6 und eines Rotors 7. Der Rotor 7 ist auf einer Antriebswelle 8 drehbar gelagert und weist Förderelemente auf, die bei Rotation das Blut in Richtung der Pfeile 9 ansaugen bzw. in Richtung der Pfeile 10 in das Blutgefäß 2 ausstoßen. Dazu weisen die Förderelemente in der gezeigten Darstellung, die die expandierte Stellung der Fluidpumpe zeigt, eine schraubenförmig angeordnete Förderschaufelfläche auf.

Auf den Aufbau des Pumpengehäuses und des Rotors wird weiter unten noch genauer eingegangen.

Die Antriebswelle 8 wird vom Antrieb 11 angetrieben, der in einem Gehäuse 12 untergebracht ist. Die Antriebselemente sind in der Fig. 1 nur schematisch dargestellt und werden weiter unten ebenfalls noch detaillierter erläutert.

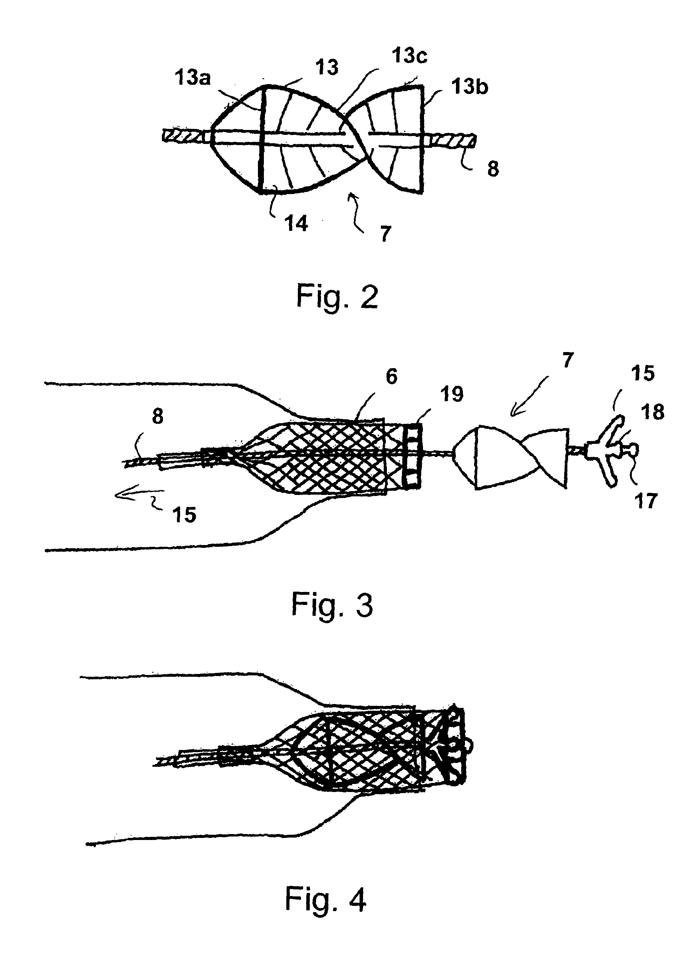

Fig. 2 zeigt im Detail einen Pumpenrotor mit zwei schraubenförmigen Förderschaufeln 13, 14, die am Umfang der Antriebswelle 8 gegeneinander um 180° versetzt sind. Die einzelnen Förderschaufeln bestehen aus Spannelementen wie beispielsweise Streben 13a, 13b sowie einem Rahmen 13c, der mit einer Membran, beispielsweise aus Polyurethan oder Polethylen bespannt ist. Der Rahmen und die Streben können beispielsweise aus einem Gedächtnismaterial bestehen, das seine Form temperaturabhängig einnimmt. Ein solcher Pumpenrotor könnte dann in komprimierter Form mit einer ersten Temperatur, vorzugsweise gekühlt, in den Patientenkörper eingebracht werden und sich dort nach Erwärmung auf die Körpertemperatur oder nachfolgender weiterer Erwärmung selbständig entfalten.

Es ist jedoch auch denkbar, den Pumpenrotor durch einen Drehbetrieb in Betriebsrichtung selbsttätig aufzustellen, indem sich das zu fördernde Fluid, also in dem Beispielfall Blut, in der Förderschaufel fängt und zu einer Aufrichtung der Förderschaufel durch den Fluidgegendruck führt.

Die Bauform des Rotors kann auch von dem oben Beschriebenen differieren, indem teilweise klapp- oder schwenkbare Elemente verwendet werden, um eine Förderschaufelfläche zu bilden. Die schwenkbaren Teile können dann in komprimiertem Zustand sinnvollerweise an die Antriebswelle angeklappt sein.

Die Streben 13a, 13b sowie der Rahmen 13c des Pumpenrotors sind sinnvollerweise bespannt, jedoch verläuft der Rahmen 13c in Richtung des Pumpengehäuses noch ein Stück weit über die Bespannung hinaus und bildet dort eine Einlaufschräge, die der einfacheren Verschiebung des Rotors in das Pumpengehäuse hinein dient.

Fig. 3 zeigt ein Pumpengehäuse 6, einen Rotor 7 sowie eine Lageranordnung 15, die voneinander beabstandet axial auf der Antriebswelle 8 verteilt sind. Dieser Zustand bleibt zumindest so lange erhalten, bis die genannten Teile innerhalb eines Patientenkörpers an Ort und Stelle gebracht sind.

Danach kann die Antriebswelle 8 in Richtung des Pfeils 15 zurückgezogen werden, um durch eine relative Verschiebung von Rotor, Pumpengehäuse und Lageranordnung eine funktionsfähige Baueinheit einer Fluidpumpe zu bilden.

Beim Zurückziehen der Antriebswelle 8 stößt zunächst der Mitnehmer 17 gegen die Nabe 18 der Lageranordnung 15. Beim weiteren Zurückziehen der Welle wird die Lageranordnung mitgenommen und gegen den Rotor 7 gedrückt. Dieser wird ebenfalls mitgenommen und beim weiteren Zurückziehen der Antriebswelle 8 in das Innere des Pumpengehäuses 6 hineingezogen. Dabei bewegt sich der Rotor 7 so weit in das Pumpengehäuse hinein, bis dass er vollständig durch dieses abgedeckt ist.

Das Pumpengehäuse 6 trägt an seinem offenen Ende einen Fixierring 19, in dem sich die Streben der Lageranordnung 15 verklemmen können.

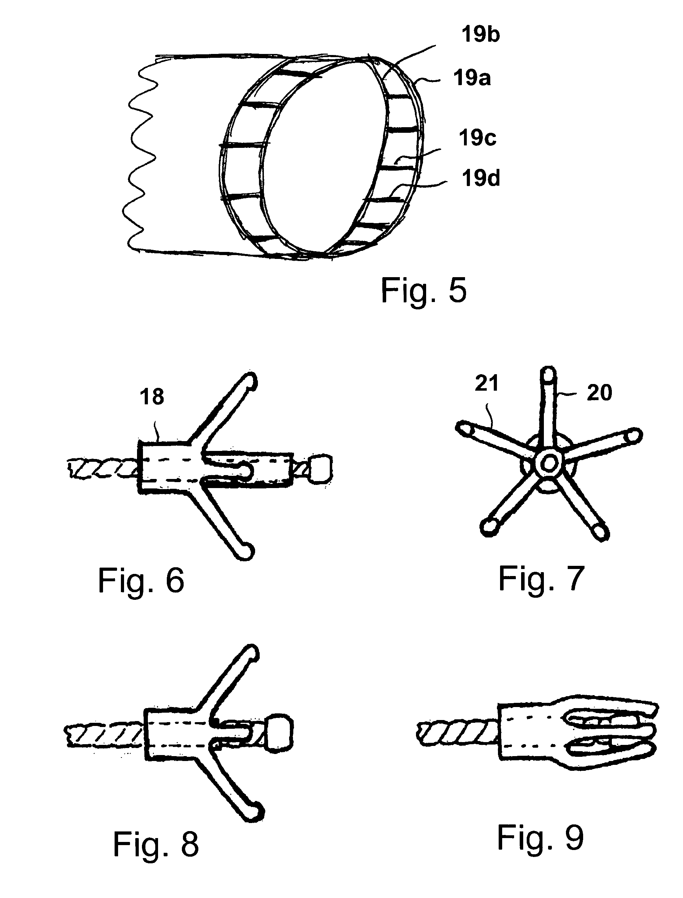

Figur 4 zeigt das Pumpengehäuse, den Rotor und die Lageranordnung in axial zusammen geschobener Form. Der Fixierring 19 ist in der Fig. 5 näher beschrieben. Er besteht aus zwei Einzelringen 19a, 19b, die auch koaxial zueinander positioniert und mittels leitersprossenartiger Verbindungsholme verbunden sind. Zwischen den Verbindungsholmen 19c, 19d finden jeweils die Enden der Streben 20,21 der Lageranordnung 15 Platz, so dass nach dem Einführen die Lageranordnung dort sowohl radial zentriert als auch axial gegenüber dem Ring 19 positioniert ist. Die Lageranordnung ist schematisch in Fig. 6 dargestellt und weist eine Nabe 18 und Streben 20, 21 auf. Die Streben 20, 21 sind an der Nabe 18 entweder mittels eines Gelenks oder durch ihre Biegsamkeit schwenkbar beweglich befestigt.

Wird die Lageranordnung durch Zurückziehen der Antriebswelle gegenüber dem Pumpengehäuse verschoben, so legen sich die Enden der Streben 20, 21 elastisch in die Fächer zwischen den Holmen 19c, 19d des Ringes 19. Dort verspannt sich die Lageranordnung selbsttätig und zentriert die in seiner Nabe gelagerte Antriebswelle gegenüber dem Pumpengehäuse 6.

Die Fig. 6 zeigt eine lange Lageranordnung, bei der sowohl vor als auch axial hinter den Streben jeweils ein beträchtlich langes Nabenstück angeordnet ist. Die Lageranordnung kann auch kürzer gebaut werden, indem nur auf einer Seite der Streben eine Nabe vorgesehen ist, wie in der Fig. 8 dargestellt.

Figur 7 zeigt eine Frontansicht der Lageranordnung aus Figur 6

Die Fig. 9 zeigt die Beweglichkeit der Streben im komprimierten Zustand.

Beim Einführen der Fluidpumpeneinrichtung durch ein Blutgefäß in einen menschlichen Körper werden die Streben 20, 21 zunächst eng an dem Nabenkörper anliegen, solange die Lageranordnung sich noch innerhalb des Gefäßes befindet, und dann elastisch aufgeweitet werden. Diese Elastizität sorgt nach dem Expandieren und dem axialen Zusammenziehen der Pumpenelemente dafür, dass die Lageranordnung in dem Ring 19 des Pumpengehäuses fixiert bleibt.

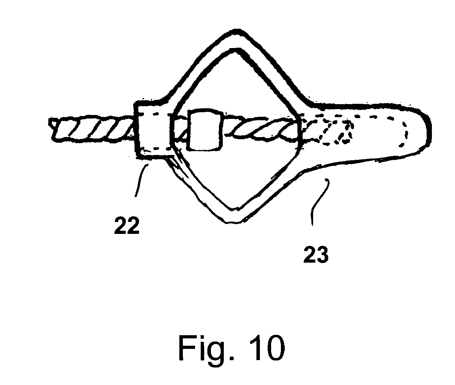

Fig. 10 zeigt eine weitere Ausführungsform der Lageranordnung mit einer doppelten Wellenlagerung, und zwar im Bereich der Lager 22, 23. Die Streben werden dadurch gebildet und radial ausgefahren, dass zwei Nabenteile 22, 23 der Lageranordnung durch axialen Zug auf die Antriebswelle einander angenähert werden. Die Streben werden bei Annäherung der beiden Nabenteile aneinander wulstartig aufgefaltet und erstrecken sich radial von der Antriebswelle weg. Bei ausreichender Expansion können auch diese Streben sich in dem Ring 19 verspannen.

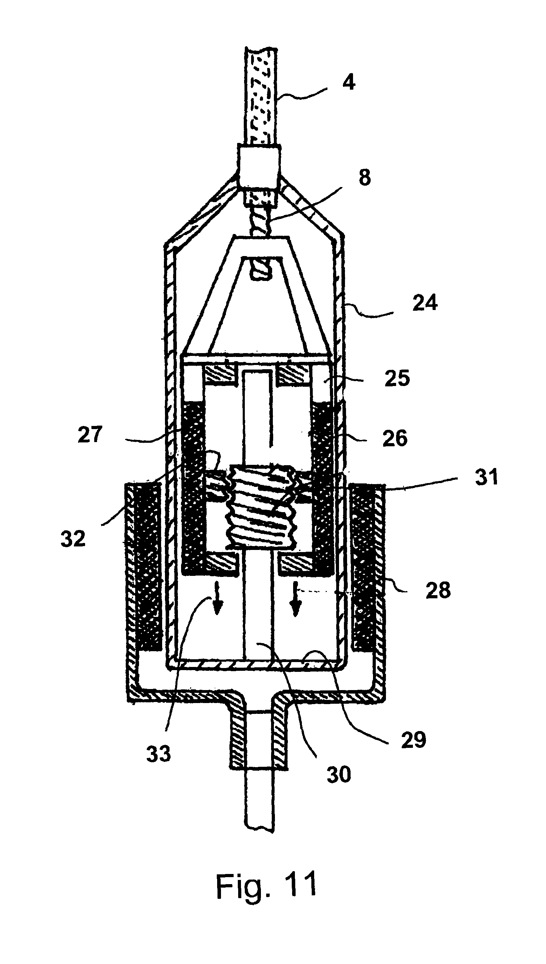

Fig. 11 zeigt das proximale Ende der Welle 8 und seine Ankopplung an einen Antrieb, die eine axiale Verschiebbarkeit der Antriebswelle um 10 bis 14 mm ermöglichen soll, um nach dem Einbringen der einzelnen Elemente der Fluidpumpe an den Einsatzort durch Zurückziehen der Antriebswelle die Lageranordnung und den Rotor in das Pumpengehäuse zurückziehen zu können.

Fig. 11 stellt in diesem Zusammenhang eine gasdichte Ankopplung des Katheters 4, der die Hülle für die Welle 8 bildet, an ein fluiddichtes Gehäuse 24 dar. In dem Gehäuse 24 befindet sich ein Antriebskörper 25, der an seinem Umfang Magnetelemente 26, 27 aufweist, welche in einem von außerhalb des Gehäuses 24 aufgebrachten veränderlichen Magnetfeld mantelseitig antreibbar sind. Dadurch wird durch die fluiddichte Wand des Gehäuses 24 in einfacher Weise eine Antriebsbewegung übertragen. Beispielsweise kann sich außerhalb des Gehäuses 24 ein zweiter Antriebskörper 28 mit Permanent- oder Elektromagneten drehen, oder es können dort Wicklungen angeordnet sein, die ein rotierendes Feld erzeugen.

Am Boden 29 des Gehäuses 24 ist ein Bolzen 30 befestigt, der ein Gewinde 31 trägt, das in axialer Richtung ortsfest ist.

Der Rotor 25 trägt einen Ring 32 mit einem Innengewinde, das auf dem Gewinde 31 läuft.

Bei Rotation des Antriebskörpers 25 in Betriebsrichtung wird durch das Zusammenwirken von Innen- und Außengewinde der Antriebskörper 25 in Richtung des Pfeils 33 bewegt, wodurch die Antriebswelle 8 zurückgezogen wird. Der Innenring 32 läuft nach Vollzug der Rückzugsbewegung der Welle von dem Gewinde 31 ab, und in der Folge kann der Antriebskörper 25 axial ortsfest rotieren. Die Fluidpumpe ist damit axial zusammengeschoben und in Betrieb gesetzt.

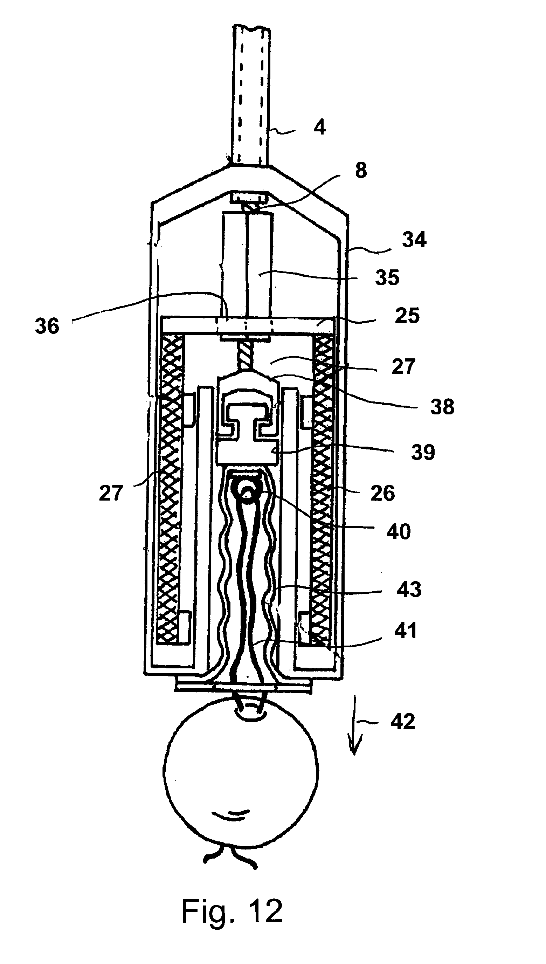

Fig. 12 zeigt eine andere Ausführungsform am proximalen Ende der Antriebswelle 8. In der Darstellung der Fig. 12 sind nur die Magnete 26, 27 des Antriebskörper 25 dargestellt, die sich innerhalb eines fluiddichten Gehäuses 34 befinden. Der Antriebskörper 25 kann in dem Gehäuse 34 axial ortsfest gelagert sein und wird von außen rotatorisch angetrieben. Er überträgt die Rotationsbewegung auf einen Mehrkant 35, der in einer komplementär geformten Öffnung (Schloss)36 in dem Antriebskörper gegenüber diesem drehfest, aber axial verschiebbar geführt ist. Beispielweise können das Schloss 36 und der Mehrkant 35 als regelmäßige Achtkante, Vierkante oder Sechskante ausgebildet sein.

Die Antriebswelle 8 ist in ihrem Endbereich 37 mit einer Buchse 38 drehfest verbunden. In der Buchse 38 ist mit einer Hinterschneidung drehbar ein Anker 39 aufgenommen, der sich nicht mit der Buchse 38 mitdreht, diese jedoch axial fixiert. Der Anker 39 ist mit einem Ring 40 versehen, durch den ein Zugband 41 gezogen ist. Wird der Anker 39 mittels des Zugbandes 41 manuell in Richtung des Pfeiles 42 zurückgezogen, so zieht der Anker die Buchse 38, die sich gegenüber dem Anker drehen kann, in Richtung des Pfeiles 42 ein Stück weit aus dem Katheter 4 heraus, so dass im Bereich der Fluidpumpe die notwendigen Verschiebungen vorgenommen werden. Der Antriebskörper 25 kann währenddessen angetrieben werden, ohne die Zugbewegung zu stören.

Um eine Dichtigkeit des Gehäuses 34 zu gewährleisten, kann der Anker 39 mittels eines Faltenbalges oder einer dicht angeschlossenen Membran 43 fluiddicht an dem Gehäuse 34 befestigt sein.

Durch die Erfindung wird somit eine effiziente Art des Antriebs mit einer Verschiebbarkeit der Antriebswelle bereitgestellt, wobei die Verschiebung der Antriebswelle in optimierter Art zur Fertigstellung der Fluidpumpe nach dem Einbringen an den Betriebsort durch eine relative Axialverschiebung von Rotor, Lageranordnung und Pumpengehäuse genutzt wird.

## Patentansprüche

1. Im Durchmesser veränderbare Fluidpumpeneinrichtung, insbesondere für die medizinische Verwendung, mit einem im Durchmesser veränderbaren Pumpengehäuse (6), einem im Durchmesser veränderbaren Rotor (7) mit wenigstens einem Förderelement (13,14) für das Fluid sowie mit mindestens einem in einer Hülle (4) verlaufenden, gegenüber dieser bewegbaren Betätigungsmittel (8), an dessen distalem Ende die Fluidpumpe (3) und an dessen proximalem Ende eine Betätigungsvorrichtung, insbesondere eine Zugvorrichtung angeordnet ist, **dadurch gekennzeichnet, dass** das Pumpengehäuse (6) und der Rotor (7) durch Betätigung des Betätigungsmittels (8) relativ zueinander verschiebbar sind.

2. Fluidpumpeneinrichtung nach Anspruch 1, **dadurch gekennzeichnet, dass** das Betätigungsmittel eine Längsrichtung aufweist, in der es verschiebbar ist und dass das Pumpengehäuse (6) und der Rotor (7) so weit in Längsrichtung des Betätigungssmittels (8) gegeneinander verschiebbar sind, dass sie in Längsrichtung hintereinander angeordnet werden können oder mit einer gegenseitigen Überlappung in Längsrichtung, die geringer ist als die Überlappung während des Betriebs der Fluidpumpe.

3. Fluidpumpeneinrichtung nach Anspruch 1 oder 2, **dadurch gekennzeichnet, dass** am distalen Ende des Betätigungsmittels (8) von der Betätigungsvorrichtung aus gesehen hinter dem Rotor (7) eine Lageranordnung (15) auf dem Betätigungsmittel (8)oder seiner Verlängerung angeordnet ist.

4. Fluidpumpeneinrichtung nach Anspruch 3, **dadurch gekennzeichnet, dass** die Lageranordnung (15) auf dem Betätigungsmittel (8)oder seiner Verlängerung in dessen/deren Längsrichtung verschiebbar ist.

5. Fluidpumpeneinrichtung nach Anspruch 3 oder 4, **dadurch gekennzeichnet, dass** die Lageranordnung (15) in Längsrichtung des Betätigungsmittels (8) relativ zum Rotor verschiebbar ist.

6. Fluidpumpeneinrichtung nach Anspruch 3, 4 oder 5, **dadurch gekennzeichnet, dass** das Betätigungsmittel (8)oder seine Verlängerung einen Anschlagkörper (17) trägt, der beim Zurückziehen des Betätigungsmittels in
Richtung der Betätigungsvorrichtung die Lageranordnung (15) mitnimmt.

7. Fluidpumpeneinrichtung nach Anspruch 3, 4, 5 oder 6, **dadurch gekennzeichnet, dass** die Lageranordnung (15) mit dem Rotor (7) in das Innere des Pumpengehäuse (6) bewegbar ist und dass sternförmig angeordnete Streben (20,21) vorgesehen sind, die sich zwischen einer Nabe (18) der Lageranordnung und dem Pumpengehäuse elastisch verspannen.

8. Fluidpumpeneinrichtung nach Anspruch 7, **dadurch gekennzeichnet, dass** die Streben (20,21) elastisch schwenkbar an der Nabe (18) der Lageranordnung (15) befestigt sind.

9. Fluidpumpeneinrichtung nach Anspruch 8, **dadurch gekennzeichnet, dass** zwischen zwei und acht Streben (20,21) vorgesehen sind.

10. Fluidpumpeneinrichtung nach Anspruch 7, 8 oder 9, **dadurch gekennzeichnet, dass** die Streben (20,21) im verspannten Zustand einen Einströmkäfig am distalen Ende der Fluidpumpe bilden.

11. Fluidpumpeneinrichtung nach Anspruch 1 oder einem der folgenden, **dadurch gekennzeichnet, dass** das Betätigungsmittel (8) gegenüber der Hülle (4) in Längsrichtung, insbesondere auf die Betätigungsvorrichtung zu, verschiebbar ist.

12. Fluidpumpeneinrichtung nach Anspuch 1 oder einem der folgenden, **dadurch gekennzeichnet, dass** das Betätigungsmittel eine in ihrer Längsrichtung verschiebbare Antriebswelle ist.

13. Fluidpumpeneinrichtung nach Anspuch 1 oder einem der folgenden, **dadurch gekennzeichnet, dass** die Betätigungsvorrichtung Teil eines Rotationsantriebs ist.

14. Fluidpumpeneinrichtung nach Anspruch 12 oder 13, **dadurch gekennzeichnet, dass** das antriebsseitige Ende der Antriebswelle (8) mit einem in einem dichten
Gehäuse (24,34) angeordneten, von außerhalb des Gehäuses magnetisch rotatorisch antreibbaren Antriebskörper (25) verbunden ist.

15. Fluidpumpeneinrichtung nach Anspruch 14, **dadurch gekennzeichnet, dass** der Antriebskörper (25) in Längsrichtung der Antriebswelle (8) verschiebbar ist und mantelseitig durch ein veränderliches magnetisches Feld angetrieben wird.

16. Fluidpumpeneinrichtung nach Anspruch 14, **dadurch gekennzeichnet, dass** die Antriebswelle (8) fest mit einem Mitnahmekörper (35) verbunden ist, der seinerseits in Längsrichtung der Antriebswelle (8) verschiebbar drehfest in dem Antriebskörper (25) geführt ist.

17. Verfahren zum Betrieb einer Fluidpumpeneinrichtung nach Anspruch 1 oder einem der folgenden, **dadurch gekennzeichnet, dass** die Fluidpumpeneinrichtung in komprimiertem Zustand an einen Einsatzort gebracht wird, dass danach wenigstens das Pumpengehäuse (6) wenigstens teilweise expandiert und dass danach das Pumpengehäuse und der Rotor (7) derart in Längsrichtung des Betätigungsmittels (8) gegeneinander verschoben werden, dass der Rotor, insbesondere vollständig, in dem Pumpengehäuse aufgenommen wird.
